# EUROPEAN PATENT APPLICATION

(11) **EP 1 174 108 A1**
(43) Date of publication of application: **23.01.2002**
(21) Application number: 99973627.5
(22) Date of filing: 06.12.1999
(51) Int. Cl.: A61K 7/00

(54) **COSMETICS**

(30) Priority: 20.01.1999 JP 1211599; 20.10.1999 JP 29784299
(71) Applicant: Faith Co., Ltd, Osaka-shi, Osaka 543-0002 (JP); Kojima, Tyuyoshi, Osaka-shi, Osaka 543-0036 (JP)
(72) Inventor: NISHIOKA, Hajime, Sakyo-ku, Kyoto-shi, Kyoto 606-0072 (JP)
(74) Representative: Stuart, Ian Alexander
(86) International application number: JP9906851
(87) International publication number: WO0042976

(57) **Abstract**

The object of the present invention is to provide a cosmetic primary product used in cosmetics which does not generate active oxygen. The cosmetic primary product of the present invention is produced from one or a combination of two or more of substances, from which no active oxygen is generated, such as a base, antioxidant, moisturizing agent, a neutralizing agent, a thickener, a foaming agent, an antiseptic agent, a refrigerant, a buffer, a pH adjuster, a coloring agent, an vehicle, a sponifier, a dispersing agent, an anti-inflammatory agent, an emulsifier, oil and fat, an ultraviolet absorber, a skin adjustor, a solubilizer, a surfactant, an astringent, a lubricant, an emollient, an ultraviolet ray scattering agent, and a thickening adjuvant.

## Description

### Technical Field

The present invention relates to a cosmetic primary product used in cosmetics, and in particular to a cosmetic primary product produced from only a substance or substances from which no active oxygen is generated.

### Background Art

Conventional cosmetic primary products have been produced regardless of the generation or non-generation of active oxygen from substances constituting the cosmetic primary products. Today, an adverse effect of active oxygen on human bodies is greatly taken up. In the light of this fact, measures that a substance for eliminating active oxygen or a substance for suppressing the generation of active oxygen is incorporated into cosmetic primary products are being taken.

However, cosmetic primary products are applied directly to human bodies. Therefore, the cosmetic primary products influence the human bodies directly. In the actual situation, such measures that a substance for eliminating active oxygen or a substance for suppressing the generation of active oxygen is incorporated into cosmetic primary products are not drastic solutions. Moreover, there have not yet been published any literature or the like in which it is systematically examined or discussed whether or not active oxygen is generated from raw materials of cosmetics.

In the light of the above-described situation, therefore, the inventor used Cut Sod Assay method (BIO REVIEW, Vol. 14, No. 6, 1997 by Hajime Nishioka), which is a method for detecting active oxygen in a cell, comprising the steps of preparing a deficient variant strain of an active oxygen eliminating enzyme of colon bacilli by genetic manipulation and using this strain. This method is different from the spin trap method using ROS (electron spin resonance) wherein the suppression degree of active oxygen of a chemical substance in a solution is generally measured. The inventor measured raw materials generally used in cosmetics. As a result, the inventor has selected raw materials of cosmetics from which active oxygen is not generated.

### Disclosure of Invention

Thus, from the above-described results the inventor found a production of a cosmetic primary product from only a substance or substances from which active oxygen is not generated.

The cosmetic primary product according to claim 1 of the present invention is a cosmetic primary product prepared from a substance from which no active oxygen is generated. With the use of the thus prepared cosmetic primary product, no adverse effect of active oxygen is produced.

The cosmetic primary product according to claim 2 of the present invention has the structure of claim 1, and is further prepared by selecting one or a combination of two or more of: a base from which no active oxygen is generated, an antioxidant from which no active oxygen is generated, a moisturizing agent from which no active oxygen is generated, a neutralizing agent from which no active oxygen is gencrated, a thickonor from which no active oxygen is generated, a foaming agent from which no active oxygen is generated, an antiseptic agent from which no active oxygen is generated, a refrigerant from which no active oxygen is generated. a buffer from which no active oxygen is generated, a pH adjuster from which no active oxygen is generated, a coloring agent from which no active oxygen is generated, an vehicle from which no active oxygen is generated, a sponifier from which no active oxygen is generated, a dispersing agent from which no active oxygen is generated, an anti-inflammatory agent from which no active oxygen is generated, an emulsifier from which no active oxygen is generated, oil and fat from which no active oxygen is generated, an ultraviolet absorber from which no active oxygen is generated, a skin adjuster from which no active oxygen is generated, a solubilizer from which no active oxygen is generated, a surfactant from which no active oxygen is generated, an astringent from which no active oxygen is generated, a lubricant from which no active oxygen is generated, an emollient from which no active oxygen is generated, an ultraviolet ray scattering agent from which no active oxygen is generated, and a thickening adjuvant from which no active oxygen is generated.

### Brief Description of the Drawings

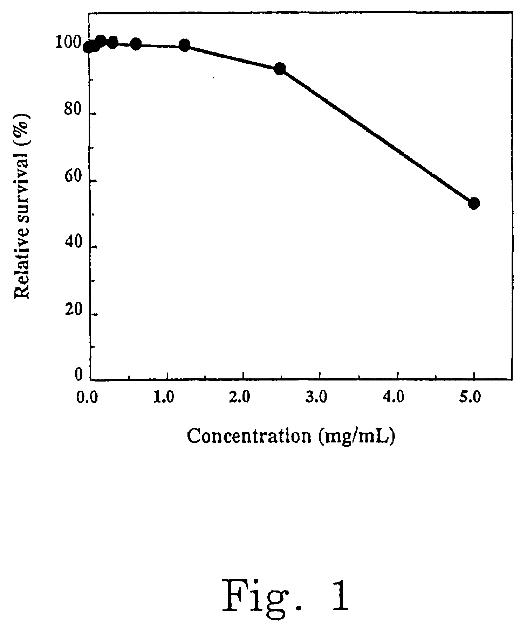

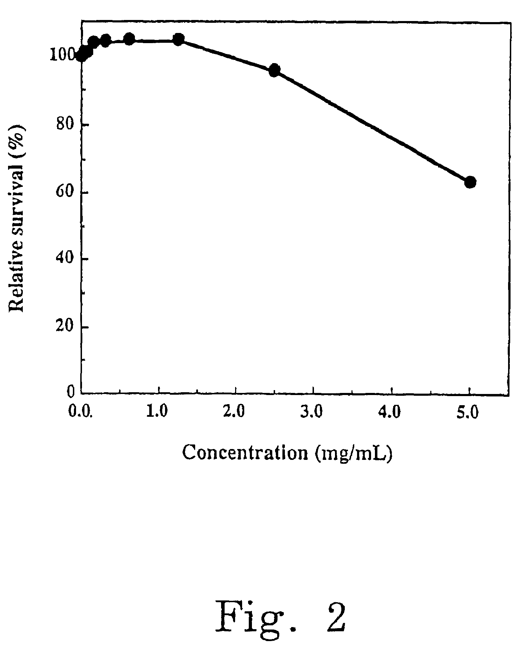

FIG. 1 is a graph showing results of a cytotoxicity test using HeLa cells of a hamamelis extracted liquid <Stock Number 202>, and FIG. 2 is a graph showing results of a cytotoxicity test using HeLa cells of a hamamelis extracted liquid (20) <Stock Number 110>.

### Best mode for Carrying out the Invention

Depending on blend-purposes, components constituting cosmetic primary products arc sorted out as follows: a base, an antioxidant, a moisturizing agent, a neutralizing agent, a thickener, a foaming agent, an antiseptic agent, a refrigerant, a buffer, a pH adjuster, a coloring agent, an vehicle, a sponifier, a dispersing agent, an anti-inflammatory agent, an emulsifier, oil and fat, an ultraviolet absorber, a skin adjuster, a solubilizer, a surfactant, an astringent, a lubricant, an emollient, an ultraviolet ray scattering agent, and a thickening adjuvant. Substances from which active oxygen is not generated are selected from these components. One or more of the substances from which active oxygen is not generated are combined and blended to prepare a cosmetic primary product from which active oxygen is not generated.

As the base, antioxidant, moisturizing agent, neutralising agent, thickener, foaming agent, antiseptic agent, refrigerant, buffer, pH adjuster, coloring agent, vehicle, sponifier, dispersing agent, anti-inflammatory agent, emulsifier, oil and fat, ultraviolet absorber, skin adjuster, solubilizer, surfactant, astringent, lubricant, emollient, ultraviolet ray scattering agent, and thickening adjuvant, each of which being a blend component of cosmetic primary products, typical examples of substances from which active oxygen is not generated will be described below. Detection experiment of active oxygen generation has proved that no active oxygen is generated from these substances. As the detection method, Cut Sod Assay method is used.As described above, in this detection method a deficient variant strain of an active oxygen eliminating enzyme of colon bacilli is prepared by genetic manipulation and then this strain is used.
Base: kaolin, adsorption-purified lanolin, purified water, modified alcohol regulated by the government, and methylpolysiloxane.
Antioxidant: disodium L-ascorbic acid sulfate, natural vitamin E, and a garlic extract.
Moisturizing agent: diglycerin, concentrated glycerin, succinyl atelocollagen solution, sodium hyaluronate (1), sodium hyaluronate (2), sodium hyaluronate (8), a placenta extract (3), a hamamelis extracted liquid (20), a chamomile extract (1), sorbit, aloe sap powder (2), a seaweed extract and halogenated soy bean phospholipid.
Neutralizing agent: L-arginine.
Thickener: carrageenan. xanthane gum, and sodium chloride.
Foaming agent: N-coconut oil fatty acid acyl ester-potassium L-glutamate and coconut fatty acid triethanolamine solution.
Antiseptic agent: a chamomile extract (1), and a garlic extract.
Tonic: ethanol.
Buffer: citric acid.
pH adjuster: citric acid and sodium hydroxide.
Coloring agent: gardenia blue.
Excipient: ethylene glycol distearate, stearic acid, myristic acid, lauric acid, aluminum magnesium silicate, and kaolin.
Sponifier: potassium hydroxide.
Dispersing agent: polyoxyethylene phytosterol (5E.0), and sorbitol monoisostearate.
Anti-inflammatory agent: dipotassium glycyrrhizate, and a saxifraga extract.
Emulsifier: sorbitol monostearate, polyoxyethylene coconut oil fatty acid glycerin ester, polyglyceryl monomyristate, polyoxyethylene hardened castor oil, lipophilic glycerin monostearate, glyceryl tri 2 - ethylhexanoate, and coconut oil fatty arid triethanol ester solution.
Oil and fat: squalane, olive oil, fluid paraffin, and jojoba oil.
Ultraviolet absorber: titanium oxide, and ultraviolet absorber-containing polymethyl methacrylate.
Skin adjuster: hamamelis extracted liquid (20).
Solubilizer: polyoxyethylene coconut oil fatty acid glycerin ester.
Surfactant: lauric acid myristic acid diethanol amide
Astringent: a chamomile extract (1).
Lubricant: squalane.
Emollient: isonoyl isonanonate.
Ultraviolet ray scattering agent: titanium oxide.
Thickening adjuvant: sodium lose and sodium carboxymethylcelluose.

### [Test examples]

Next, the inventor made the following experiments to demonstrate that the present invention is very useful. That is, it is known according to detection experiments of active oxygen generation according to the above-mentioned Cut Sodium Assay that active oxygen is generated from a hamamelis extracted liquid <Stock Number 202> made by Koei Kogyo Co., Ltd. among hamamelis extracted liquids used as a common blend component of a cosmetic primary product but no active oxygen is generated from a hamamolis extracted liquid (20) <Stock Number 410> made by Maruzen Pharmaceuticals Co., Ltd. among the same. The inventor made an experiment as to how much these hamamelis extracted liquids exhibited toxicity against cells.

### Summary of the method for the test

In order to examine cytotoxicity against HoLa cells of the hamamelis extracted liquid <Stock Number 203> and the hamamelis extracted liquid (20). <Stock Number 410>, a cytotoxicity test based on the Neutral Red method using HeLa. S3(SC) cells originating from humans was performed.In the present test, the highest concentration was set to 5 mg/mL, and eight kinds of concentrations, wherein their common ratio was 2, were set up.

As a result, the hamamelis extracted liquid <Stock Number 202> having the highest concentration of 5 mg/mm exhibited a survival rate of 53.2% relative to a solvent control. The hamamelis extracted liquid (20) <Stock Number 410> having the highest concentration of 5 mg/mL exhibited a survival rate of 63.6% relative to the solvent control. The survival rate of the hamamelis extracted liquid <Stock Number 203> was about half while that of the hamamelis extracted liquid.(20) <Stock Number 410> was about two-third.It can be understood from this fact that toxicity of the hamamelis extracted liquid (20) <Stock Number 410> was clearly less than that of the hamamelis extracted liquid <Stock Number 202>.

In general, cytotoxicity tests using a 96-well plate are simple and easy to handle. The tests have reproducibility, and make it possible to detect a cytotoxicity effect on cells in a few days. In Japan, various cytotoxicity test methods are used in validation tests for alternatives to animal experiments, and the usefulness of each of the tests is verified. In particular, the method using Neutral Red (NR) is a method wherein the characteristic that NR accumulates in lysosome in living cells is, and this method has an advantage that the number of the living cells can be accurately measured. In this method, specimens are added to cells inoculated into 96 wells, and then an NR solution is added thereto, so as to cause the NR solution to be taken in the living cells. Thereafter, NR is extracted to measure their absorbances. On the basis of the obtained measurement results, the group of the specimens and the group of controls are compared to calculate their survival rates. They are used as indexes of cytotoxicity of the specimens.

Raw materials used in the test and the method of the test are as follows.

### 1. Two specimens

(1) As to the hamamelis extracted liquid <Stock Number 202>, distilled water for injection (DW, Lot number K8L76, Otsuka Pharmaceutical Co., Ltd.) is used as a solvent, and a solution having a concentration 20 times the highest treating concentration is prepared. This solution is used as a stock solution, and is distilled with the solvent and adjusted in a medium so as to have a concentration of 10 % by volume. To each well into which 0.1 mL of a medium is put is added 0.1 mL of the adjusted solution, and this well is used (the amount of the solvent in the treating solution is 5% by volume).
(2) As to the hamamelis extracted liquid (20) <Stock Number 410>, distilled water for injection (DW. Lot number: K8L76, Otsuka Pharmaceutical Co., Ltd.) is used as a solvent, and a solution having a concentration 20 times the highest treating concentration is prepared. This solution is used as a stock solution, and is distilled with the solvent and adjusted in a medium so as to have a concentration of 10 % by volume. To each well into which 0.1 mL of a medium is put is added 0.1 mL of the adjusted solution. This is used (the amount of the solvent in the treating solution is 5% by volume).

### 2. Cells

HeLa. S3(SC) cells (HeLa cells, which are cells used in validation tests sponsored by Japanese Society of Alternatives to Animal Experiments) cultivated in a C0₂ incubator (CO2 concentration: 5%.37 °C ) using Eagle's MEM medium containing 10% by volume of calf serum (Cansera International. Lot number: 28110754) was used.

### 3. Positive control material

In order to check whether or not cell propagation is suppressed dependently on cytotoxicity effect of the specimens, lauryl sulfate sodium salt (SDS, Sigma Chemical Co., Ltd. Lot number: 115H0347), about which it is known to exhibit cytotoxicity is used, as a positive control material. Treating concentrations are 4 kinds of 25 µ g/ml, 50µ g/mL, 75µ g/mL and 100µ g/mL. A solution obtained by adjusting a solution having a concentration 20 times the treating concentration with distilled water for injection is used as a stock solution. The same treating method as for the above-mentioned specimens is performed.

### 4. Method of the test

As to the treating concentrations, the highest concentrations for the two specimens are set to 5 mg/mL and 8 kinds of concentrations (0.0391 mg/mL, 0.0781 mg/mL, 0.156 mg/mL, 0.313 mg/mL, 0.625 mg/mL, 1.25 mg/mL, 2.5 mg/mL and 5 mg/mL), wherein their common ratio is 2, are set up. Outermost, wells of 96-well plates are not used for the test, and 0.1 mL of PBS(-) is added to each well. The medium is added to each blank well in an amount of 0.1 mL/well. HeLa cells which are in their logarithmic growth phase are prepared into a suspension having a cell concentration of 4 × 104 cells/mL, using the medium. Into each of the wells other than the blank wells is poured 0.1 mL (3 × 103 cells) of the cell suspension. Four wells for each of the concentrations are used per plato. Three plates are prepared per specimen. On the next day, to each of the wells is added 0.1 mL of each of the specimens prepared in the medium and the positive control material, so that they have a defined final concentration. To each of the wells is added 0.1 mL of a solvent control group prepared in the medium so as to have a solvent concentration of 10% by volume (final concentrations: 5% by volume). To each of blank wells is added 0.1 mL of only the medium The respective plates are further cultivated. After two days, to each of the wells including the blank wells is added 0.1 mL of the NR solution (150 µ g/mL medium). The resultant is cultivated for 2 hours so as to cause the colorant to be taken in the cells. After the end of the cultivation, the medium is removed and then a fixing solution (1% CaCl2-1% aqueous formalin solution) is added thereto in an amount of 150 µ L/well and then fixed for about 1 minute. Thereafter, the fixing solution is sufficiently removed and then an extracting solution (1% acetic acid-50% aqueous ethanol solution) is added thereto in an amount of 100 µ L/well. Each of the plates is allowed to stand still at room temperature for about 20 minutes to extract the colorant. The plate is lightly shaken to extract the colorant uniformly. Thereafter, a plate reader is used to measure the absorbance at a wavelength of 540 nm.

### 5. Calculating method

For each of the plates; the average of absorbances at each of the concentrations is obtained. Furthermore, for each of the concentrations, the average of absorbances in the three plates is obtained. The value that the value of the blank is subtracted from each of the absorbance groups is used to obtain the survival rate of each of the treated groups relative to the solvent control group.

### 6. Test results

Both of the hamamelis)is extracted liquid <Stock Number 202> and the hamamelis extracted liquid (20) <Stock Number 410> are dark brown solutions, and are completely miscible with the DW (distilled water for injection) and the medium. Thus, even at the highest treating concentration of 5 mg/mL, no precipitations were generated.

As shown in FIG. 1 and Table 1, the hamamelis extracted liquid <Stock Number 202> *did* not exhibit toxicity effect on the HeLa cells at the concentrations of 2.5 Mg/mL or less (relative survival rate: 90% or more). However, the hamamelis extracted liquid <Stock Number 202> exhibited *a* relative survival rate of 53.2 % at the concentration of 5 mg/mL, as compared with the solvent control.

As shown in FIG. 2 and Table 2, the hamamelis extracted liquid (20) <Stock Number 410> did not exhibit toxicity effect on the HeLa cells at the concentrations of 2.5 mg/mL or less (relative survival rate: 95% or more). However, the hamamelis extracted liquid <Stock Number 202> exhibited a relative survival rate of 63.6 % at the concentration of 5 mg/mL, as compared with the solvent control.

The SDS used as the positive control exhibited IC50 at 35-4 µg/mL in the test of the hamamelis extracted liquid <Stock Number 202>. as shown in Table 1. The SDS exhibited IC50 at 35.6 µg/mL in the test of the hamamelis extracted liquid (20) <Stock Number 410>, as shown in Table 2.

As is understood from the above-mentioned results, the hamamelis extracted liquid <Stock Number 202> and the hamamelis extracted liquid (20) <Stock Number 410> exhibited survival rates of 53.2% and 63.6% relative to the solvent control, respectively, at the highest concentration of 5 mg/mL under the above-mentioned lest conditions. Therefore, it was found out that the hamamelis extracted liquid (20) <Stock Number 410> has far lower toxicity and higher safety than the hamamelis extracted liquid <Stock Number 202>

**[Table 1]**

| Results of the cytotoxicity test of the hamamelis extracted liquid <Stock Number 202>, using HeLa cells | | | | | | |
|---|---|---|---|---|---|---|
| Concentration (mg/mL) | OD₅₄₀ (the average of 4 wells/plate) | | | Average±S.D. (true absorbance*)(%) | Relative survival rate | IC₅₀ |
| | 1 | 2 | 3 | | | |
| Blank | 0.036 | 0.028 | 0.026 | 0.030 ±0.005 (0.000) | 0.0 | |
| Solvent control | 0.470 | 0.473 | 0.472 | 0.472 ±0.002 (0.442) | 100.0 | |
| 0.0391 | 0.469 | 0.473 | 0.480 | 0.474 ±0.006 (0.444) | 100.5 | ≥ 5mg/mL |
| 0.0781 | 0.473 | 0.497 | 0.454 | 0.474 ±0.021 (0.444) | 100.5 | |
| 0.156 | 0.481 | 0.482 | 0.479 | 0.480 ±0.001 (0.450) | 101.8 | |
| 0.313 | 0.484 | 0.493 | 0.455 | 0.477 ±0.020 (0.447) | 101.1 | |
| 0.625 | 0.477 | 0.494 | 0.457 | 0.476 ±0.018 (0.446) | 100.9 | |
| 1.25 | 0.471 | 0.481 | 0.466 | 0.473 ±0.008 (0.443) | 100.2 | |
| 2.5 | 0.444 | 0.460 | 0.422 | 0.442 ±0.019 (0.412) | 93.2 | |
| 5 | 0.270 | 0.282 | 0.244 | 0.265 ±0.019 (0.235) | 53.2 | |
| | | | | | | |

| SDS(µ g/mL) | | | | | | |
|---|---|---|---|---|---|---|
| 25 | 0.409 | 0.395 | 0.411 performed. | 0.405 ±0.009 (0.375) | 84.8 | |
| 50 | 0.044 | 0.033 | 0.081 | 0.036 ±0.007 (0.007) | 1.4 | 35.4µg/mL |
| 75 | 0.032 | 0.029 | 0.027 | 0.029 ±0.003 (.0.001) | -0.2 | |
| 100 | 0.034 | 0.031 | 0.026 | 0.030 ±0.004 (0.000) | 0.0 | |

| | | | | | | |
|---|---|---|---|---|---|---|
| *: Average absorbance at each concentration in three plates - Average absorbance of blanks in the three plates | | | | | | |

**[Table 2]**

| Results of the cytotoxicity test of the hamamelis extracted liquid (20) <Stock Number 410>, using HeLa cells | | | | | | |
|---|---|---|---|---|---|---|
| Concentration (mg/mL) | OD₅₄₀ (the average of 4 wells/plate) | | | Average±S.D. (true absorbance*)(%) | Relative survival rate | IC₅₂₀ |
| | 1 | 2 | 3 | | | |
| Blank | 0.033 | 0.024 | 0.031 | 0.029 ±0.005 (0.000) | 0.0 | |
| Solvent control | 0.484 | 0.516 | 0.505 | 0.501 ±0.016 (0.472) | 100.0 | |
| 0.0391 | 0.498 | 0.508 | 0.514 | 0.507 ±0.008 (0.478) | 101.3 | >5mg/mL |
| 0.0781 | 0.501 | 0.502 | 0.516 | 0.506 ±0.008 (0.477) | 101.1 | |
| 0.156 | 0.512 | 0.505 | 0.537 | 0.518 ±0.017 (0.489) | 103.6 | |
| 0.313 | 0.530 | 0.494 | 0.542 | 0.522 ±0.025 (0.493) | 104.4 | |
| 0.625 | 0.530 | 0.510 | 0.532 | 0.524 +0.012 (0.495) | 104.9 | |
| 1.25 | 0.526 | 0.513 | 0.531 | 0.523 ±0.009 (0.494) | 104.7 | |
| 2.5 | 0.473 | 0.478 | 0.496 | 0.482 ±0.012(0.453) | 96-0 | |
| 5 | 0.327 | 0.339 | 0.322 | 0.329 ±0.009 (0.300) | 63.6 | |
| | | | | | | |

| SDS(µ g/mL) | | | | | | |
|---|---|---|---|---|---|---|
| 25 | 0.434 | 0.430 | 0.445 | 0.436 ±0.008 (0.407) | 86.2 | |
| 50 | 0.036 | 0.025 | 0.034 | 0.032 ±0.007 (0.003) | 0.6 | 35.6 µ g/mL |
| 75 | 0.031 | 0.021 | 0.030 | 0.027 ±0-005 (-0.002) | -0.4 | |
| 100 | 0.039 | 0.022 | 0.029 | 0.030 ±0.009 (0.001) | 0.2 | |

| | | | | | | |
|---|---|---|---|---|---|---|
| performed. *: Average absorbance at each concentration in three plates - Average absorbance of blanks in the three plates | | | | | | |

The above-mentioned test is one example. It can be said from this test example that if a certain specific cosmetic primary product contains, in its components, the hamamolis extracted liquid <Stock Number 202> from which active oxygen is generated and no active oxygen is generated from components other than the hamamelis extracted liquid <Stock Number 202>, the cosmetic primary product contains a substance harmful to human bodies. This is because the hamamelis extracted liquid <Stock Number 202> can be recognized to have toxicity as shown in the above-mentioned test results. Thus, in the case that the hamamelis extracted liquid <Stock Number 202> is replaced by the hamamelis extracted liquid (20) <Stock Number 410>, which is a substitution substance, so as to prepare a cosmetic primary product comprising as components only substances from which no active oxygen is generated as in the present invention, no toxicity is generated by active oxygen. In reality, many substances from which active oxygen is generated are incorporated into components of an ordinary cosmetic primary product. In order to produce the cosmetic primary product of the present invention, it is verified that active oxygen is not generated from performed. its respective components as demonstrated in the present example. Thereafter, the cosmetic primary product is prepared.

The experimental examples of cosmetic primary products prepared by using the above-mentioned substances from which no active oxygen is generated will be described below.

### [First experimental example: production of cosmetic water]

Cosmetic water was produced using the following substances, from which no active oxygen is generated, in the following blend ratios. As a result, it was proved that the produced water had a good quality as cosmetic water and no active oxygen was generated.

| Blend component | Blend ratio (%) | Purpose of blend |
|---|---|---|
| Concentrated glycerin | 8.00 | moisturizing agent |
| Diglycerin | 4.00 | moisturizing agent |
| Modified alcoholregulated by the government | 5.00 | base |
| Dipotassium glycyrrhizate | 0.10 | anti-inflammatory agent |
| Sodium hyaluronate (1) | 0.02 | moisturizing agent |
| Placenta extract (3) | 0.30 | moisturizing agent |
| Saxifraga extra | 0.20 | anti-inflammatory agent |
| Chamomile extract (1) | 0.50 | antiseptic agent,astringent and moisturizing agent |
| Garlic extract | | antiseptic agent and antioxidant |
| Purified water | 81.38 | base |

### [Second experimental example: production of milky lotion]

Milky lotion was produced using the following substances, from which no active oxygen is generated, in the following blend ratios. As a result, it was proved that the produced lotion had a good quality as milky lotion and no active oxygen was generated.

| Blend component | Blend ratio (%) | Purpose of blend |
|---|---|---|
| Squalane | 1.00 | oil and fat, andlubricant |
| Stearic acid | 1.50 | vehicle |
| Polyoxyethylene coconut oil fatty acid glycerin eater | 0.30 | emulsifier |
| Seryl | 0.40 | emulsifier |
| Concentrated glycerin | 6.00 | moisturizing agent |
| Sorbit | 0.20 | moisturizing agent |
| Dipotassium glycyrrhizate | 0.10 | anti-inflammatory agent |
| Sodium hyaluronate (1) | 0.03 | moisturizing agent |
| Saxifraga extract | 0.20 | anti-inflammtory agent |
| Chamomile extract (1) | 0.50 | antiseptic agent, astringent and moisturizing agent |
| Garlic extract | 0.50 | antiseptic agent and antioxidant |
| Purified water | 90.27 | base |

### [Third experimental example: production of cosmetic cream]

Cosmetic cream was produced using the following substances, from which no active oxygen is generated, in the following blend ratios. As a result, it was proved that the produced cream had a good quality as cosmetic cream and no active oxygen was generated.

| Blend component | Blend ratio (%) | Purpose of blend |
|---|---|---|
| Hydrogenated soy bean phospholipid | 2.00 | moisturizing agent |
| Stearic acid | 0.50 | vehicle |
| Myristic acid | 1.20 | vehicle |
| Squalane | 6.40 | oil and fat, and lubricant |
| Isononyl isonanonato | 2.50 | emollient |
| Polyoxyethylene hardened castor oil | 1.20 | emulsifier |
| Concentrated glycerin | 10.00 | moisturizing agent |
| Dipotassium glycyrrhizate | 0.10 | anti-inflammatory agent |
| Sodium hyaluronate (3) | 0.02 | moisturizing agent |
| Placenta extract (3) | 0.50 | moisturizing agent |
| Saxifraga extract | 0.20 | anti-inflammatory agent |
| Chamomile extract (1) | 0.50 | antiseptic agent,astringent and moisturizing agent |
| Garlic extract performed. | 0.50 | antiseptic agent and antioxidant |
| Purified water | 74.38 | base |

### (Fourth experimental example: production of cleansing cream]

Cleansing cream was produced using the following substances, from which no active oxygen is generated, in the following blend ratios. As a result, it was proved that the produced cream had a good quality as cleansing cream and no active oxygen was generated.

| Blend component | Blend ratio (%) | Purpose of blend |
|---|---|---|
| Fluid paraffin | 40.00 | oil and fat |
| Stearic acid | 2.00 | vehicle |
| Lipophilic | | |
| glycerin monostearate | 1.50 | emulsifier |
| Glyceryl tri2-ethylbexanoate | 0.60 | emulsifier |
| Coconut oil fatty acid | | |
| triethanol ester liquid | 1.60 | emulsifier |
| Absorption-purified lanolin | 3.00 | base |
| Methylpolysiloxane | 0.30 | base |
| Concentrated glycerin | 8.00 | moisturizing agent |
| Diglycorin | 2.00 | moisturizing agent |
| Chamomile extract (1) | 0.50 | antiseptic agent astringent and moisturizing agent |
| Garlic extract | 0.50 | antiseptic agent and antioxidant |
| Saxifraga extract | 0.20 | anti-inflammatoryagent |
| Dipotassium glycyrrhizate | 0.10 | anti-inflammatory agent |
| Purified water | 39.70 | base |

### [Fifth experimental example: production of cleansing foam]

Cleansing foam was produced using the following substances, from which no active oxygen is generated, in the following blend ratios. As a result, it was proved that the produced foam had a good quality as cleansing foam and no active oxygen was generated.

| Blend component | Blend ratio (%) | Purpose of blend |
|---|---|---|
| Lauric acid | 7.00 | vehicle |
| Stearic acid | 15.00 | vehicle |
| Myristic acid | 17.00 | vehicle |
| Coconut oil fatty acid | | |
| triethanol ester liquid | 8.00 | emulsifier |
| Sodium hydroxide | 1.00 | pH adjuster |
| Potassium hydroxide | 7.00 | sponifier |
| Concentrated glycerin | 7.00 | moisturizing agent |
| Dipotassium glycyrrhizate | 0.20 | anti-inflammatory agent |
| Chamomile extract (1) | 0.50 | antiseptic agent,astringent and moisturizing agent |
| Garlic extract | 0.50 | antiseptic agent and antioxidant |
| Purified water | 36.80 | base |

### [Sixth experimental example: production of an agent for a cosmetic pack

An agent for a cosmetic pack was produced using the following substances, from which no active oxygen is generated, in the following blend ratios. As a result, it was proved that the produced agent had a good quality as an agent for a cosmetic pack and no active oxygen was generated.

| Blend component | Blend ratio (%) | Purpose of blend |
|---|---|---|
| Aluminum magnesium silicate | 5.00 | vehicle |
| Titanium oxide | 1.00 | ultraviolet ray scattering agent |
| Xanthane gum | 0.20 | Thickener |
| Glyceryl tri2-ethylhexanoate | 0.20 | emulsifier |
| Sodium hyaluronate (1) performed. | 0.02 | moisturizing agent |
| Concentrated glycerin | 13.00 | moisturizing agent |
| Dipotassium glycyrrhizate | 0.20 | anti-inflammatory agent |
| Chamomile extract (1) | 0.50 | antiseptic agent,astringent and moisturizing agent |
| Modified alcohol regulated by the government | 5.00 | base |
| Garlic extract | 0.50 | antiseptic agent and antioxidant |
| Purified water | 74.38 | base |

### [Seventh experimental example: production of creamy gel]

Creamy gel was produced using the following substances, from which no active oxygen is generated, in the following blend ratios. As a result, it was proved that the produced gel had a good quality as creamy gel and no active oxygen was generated.

| Blend component | Blend ratio (%) | Purpose of blend |
|---|---|---|
| Jojoba oil | 2.00 | oil and fat |
| Squalane | 2.00 | oil and fat |
| Polyoxyethylene performed. coconut oil fatty acid glycerin ester | 1.50 | emulsifier |
| Sodium carboxymethyl cellulose | 0.80 | thickening adjuvant |
| Potassium hydroxide | 0.10 | sponifier |
| Concentrated glycerin | 10.00 | moisturizing agent |
| Methylpolysiloxane | 2.00 | base |
| Sodium hyaluronate (1) | 0.02 | moisturizing agent |
| Dipotassium glycyrrhizate | 0.02 | anti-inflammaory agent |
| Chamomile extract (1) | 0.50 | antiseptic agent,astringent and moisturizing agent |
| Garlic extract | 0.50 | antiseptic agent and antioxidant |
| Purified water | 80.56 | base |

### [Eighth experimental example: production of gel for the body]

Gel for the body was produced using the following: substances, from which no active oxygen is generated, in the following blend ratios. As a result, it was proved that the produced gel had a good quality as gel for the body and no active oxygen was generated.

| Blend component performed. | Blend ratio (%) | Purpose of blond |
|---|---|---|
| Aluminum magnesium silicate | 3.00 | vehicle |
| Xanthane gum | 0.50 | thickener |
| Carragecnan | 0.20 | thickener |
| kaolin | 8.00 | vehicle |
| Dipotassium glycyrrhizate | 0.10 | anti-inflammatory agent |
| Sodium hyaluronate (1) | 0.02 | moisturizing agent |
| Concentrated glycerin | 8.00 | moisturizing agent |
| Aloe sap powder | 0.20 | moisturizing agent |
| Seaweed extract | 0.20 | moisturizing agent |
| Chamomile extract (1) | 0.50 | antiseptic agent,astringent and moisturizing agent |
| Garlic extract | 0.50 | antiseptic agent and antioxidant |
| Purified water | 78.78 | base |

### [Ninth experimental example: production of gel for the body]

Gel for the body was produced using the following substances, from which no active oxygen is generated, in the following blend ratios. As a result, it was proved that the produced gel had a good quality as gel for the body and no active oxygen was generated.

| Blend component | Blend ratio (%) | Purpose of blend |
|---|---|---|
| Aluminium magnesium silicate | 3.00 | vehicle |
| Xanthane gum | 0.50 | thickener |
| Carrageenan | 0.20 | thickener |
| kaolin | 8.00 | vehicle |
| Dipotassium glycyrrhizate | 0.10 | anti-inflammatory agent |
| Sodium hyaluronate (1) | 0.02 | moisturizing agent |
| Concentrated glycerin | 8.00 | moisturizing agent |
| Hamamelis extracted liquid (20) | 0.02 | moisturizing agent |
| Seaweed extract | 0.20 | moisturizing agent |
| Chamomile extract (1) | 0.50 | antiseptic agent,astringent and moisturizing agent |
| Garlic extract | 0.50 | antiseptic agent and antioxidant |
| Purified water | 78.78 | base |

As shown by the specific components and numerical values, in the above-mentioned experimental examples, cosmetic primary products keeping sufficient quality as cosmetics and generating no active oxygen was obtained.

Concerning cosmetic primary products, people have been hitherto making efforts to remove effects of toxicity resulting from the generation of active oxygen on human belies. The inventor repeated researches which intervene up to a level of components of cosmetic primary products. As a result, the present invention has been made. The present invention is an epoch-making invention making it possible to completely remove the effects of toxicity resulting from the generation of active oxygen on human bodies.

All of the cosmetic primary products prepared in the above-mentioned experimental examples contained a base. However, the present invention can be prepared as a cosmetic primary product that does not contain any base.

### Industrial Applicability

As specifically described above, the present invention is a cosmetic primary product prepared from a material from which no active oxygen is generated. By using this cosmetic primary product, an adverse effect of active oxygen on human bodies is complete removed.

## Claims

1. A cosmetic primary product, **characterized by** being prepared from a material from which no active oxygen is generated.

2. The cosmetic primary product according to claim 1, wherein the material from which no active oxygen is generated comprises one or a combination of two or more of: a base from which no active oxygen is generated, an antioxidant from which no active oxygen is generated, a moisturizing agent from which no active oxygen is generated, a neutralizing agent from which no active oxygen is generated, a thickener from which no active oxygen is generated, a foaming agent from which no active oxygen is generated, an antiseptic agent from which no active oxygen is generated, a refrigerant from which no active oxygen is generated, a buffer from which no active oxygen is generated, a pH adjuster from which no active oxygen is generated, a coloring agent from which no active oxygen is generated, an vehicle from which no active oxygen is generated, a sponifier from which no active oxygen is generated, a dispersing agent from which no active oxygen is generated, an anti-inflammatory agent from which no active oxygen is generated, an emulsifier from which no active oxygen is generated, oil and fat from which no active oxygen is generated, an ultraviolet absorber from which no active oxygen is generated, a skin adjuster from which no active oxygen is generated, a solubilizer from which no active oxygen is generated, a surfactant from which no active oxygen is generated, an astringent from which no active oxygen is generated, a lubricant from which no active oxygen is generated, an emollient from which no active oxygen is generated, an ultraviolet ray scattering agent from which no active oxygen is generated, and a thickening adjuvant from which no active oxygen is generated.

3. The cosmetic primary product according to claim 1 or 2, wherein the material from which no active oxygen is generated comprises one or a combination of two or more of: a base from which no active oxygen is generated, an antioxidant from which no active oxygen is generated, a moisturizing agent from which no active oxygen is generated, a neutralizing agent from which no active oxygen is generated, a thickener from which no active oxygen is generated, a foaming agent from which no active oxygen is generated, an antiseptic agent from which no active oxygen is generated, a refrigerant from which no active oxygen is generated, a buffer from which no active oxygen is generated, a pH adjuster from which, no active oxygen is generated, a coloring agent from which no active oxygen is generated, an vehicle from which no active oxygen is generated, a sponifier from which no active oxygen is generated, a dispersing agent from which no active oxygen is generated, an anti-inflammatory agent from which no active oxygen is generated, an emulsifier from which no active oxygen is generated, oil and fat from which no active oxygen is generated, an ultraviolet absorber from which no active oxygen is generated, a skin adjuster from which no active oxygen is generated, a solubilizer from which no active oxygen is generated, a surfactant from which no active oxygen is generated, an astringent from which no active oxygen is generated, a lubricant from which no active oxygen is generated, an emollient from which no active oxygen is generated, an ultraviolet ray scattering agent from which no active oxygen is generated, and a thickening adjuvant from which no active oxygen being generated, each of which is selected by using Cut Sod Assay method (B10 REVIEW. Vol. 14, No. 6,1997 by Hajime Nishioka), which is a method for detecting active oxygen in a cell, comprising the steps of preparing a deficient variant strain of an active oxygen eliminating enzyme of colon bacilli by genetic manipulation and using said strain.
